# EUROPEAN PATENT APPLICATION

(11) **EP 4 299 695 A1**
(43) Date of publication of application: **03.01.2024**
(21) Application number: 23752602.5
(22) Date of filing: 13.01.2023
(51) Int. Cl.: C10G 2/00, B01D 53/22, B01J 8/02, C07C 29/152, C07C 31/04

(54) **REACTOR**

(30) Priority: 08.02.2022 JP 2022017949
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP)
(74) Representative: Müller-Boré & Partner Patentanwälte PartG mbB
(86) International application number: PCT/JP2023/000762
(87) International publication number: WO 2023/153133

(57) **Abstract**

A reactor (1) is provided with a separation membrane (10) permeable to water vapor, a non-permeation side flow path (30) extending in an approximately vertical direction on a non-permeation side of the separate membrane (10), and a catalyst (60) configured to fill the non-permeable side flow path (30). The catalyst (60) includes a first layer (61) disposed at a lower end portion of the non-permeation side flow path (30), and a second layer (62) disposed upward of the first layer (61). A mean equivalent circle diameter of catalyst particles included in the first layer (61) is larger than a mean equivalent circle diameter of catalyst particles included in the second layer (62).

## Description

### TECHNICAL FIELD

The present invention relates to a reactor.

### BACKGROUND ART

In recent years, reactors have been developed that can improve conversion efficiency by separating a product of a conversion reaction of a raw material gas containing hydrogen and carbon oxide to a liquid fuel such as methanol and ethanol (specifically, a fuel that is in a liquid state at normal temperature and pressure).

For example, Patent Literature 1 discloses a reactor provided with a separation membrane permeable to water vapor that is one of products of the conversion reaction, a non-permeation side flow path through which a raw material gas flows, and a catalyst filling the flow path. The catalyst promotes the conversion reaction of the raw material gas to the liquid fuel.

### CITATION LIST

### Patent Literature

Patent Literature 1: JP 2018-8940A

### SUMMARY

### TECHNICAL PROBLEM

In the reactor disclosed in Patent Literature 1, the non-permeation side flow path extends in the vertical direction, and thus in a lower end portion of the non-permeation side flow path, the weight of the catalyst may act on the separation membrane, thereby damaging the separation membrane.

An object of the present invention is to provide a reactor capable of suppressing damage to a separation membrane.

### SOLUTION TO PROBLEM

A reactor according to the present invention includes a separation membrane permeable to a product of a conversion reaction of a raw material gas containing at least hydrogen and carbon oxide to a liquid fuel, a non-permeation side flow path extending in an approximately vertical direction on a non-permeation side of the separation membrane, the raw material gas flowing through the non-permeation side flow path, and a catalyst configured to fill the non-permeation side flow path and promote the conversion reaction. The catalyst includes a first layer and a second layer disposed upward of the first layer, and a mean equivalent circle diameter of catalyst particles included in the first layer is larger than a mean equivalent circle diameter of catalyst particles included in the second layer.

### ADVANTAGEOUS EFFECTS

According to the present invention, a reactor capable of suppressing damage to a separation membrane can be provided.

### BRIEF DESCRIPTION OF DRAWINGS

FIG. 1 is a cross-sectional view of a reactor according to an embodiment.
FIG. 2 is a cross-sectional view of a catalyst according to a comparative example.
FIG. 3 is a cross-sectional view of a catalyst according to a comparative example.
FIG. 4 is a cross-sectional view of a catalyst according to an example.
FIG. 5 is a cross-sectional view of a catalyst according to an example.
FIG. 6 is a cross-sectional view of a reactor according to Modification 2.
FIG. 7 is a cross-sectional view of the reactor according to Modification 2.

### DESCRIPTION OF EMBODIMENTS

Embodiments of the present invention will be described with reference to the drawings. However, the drawings are schematic, and ratio or the like of dimensions may differ from an actual one.

### Reactor 1

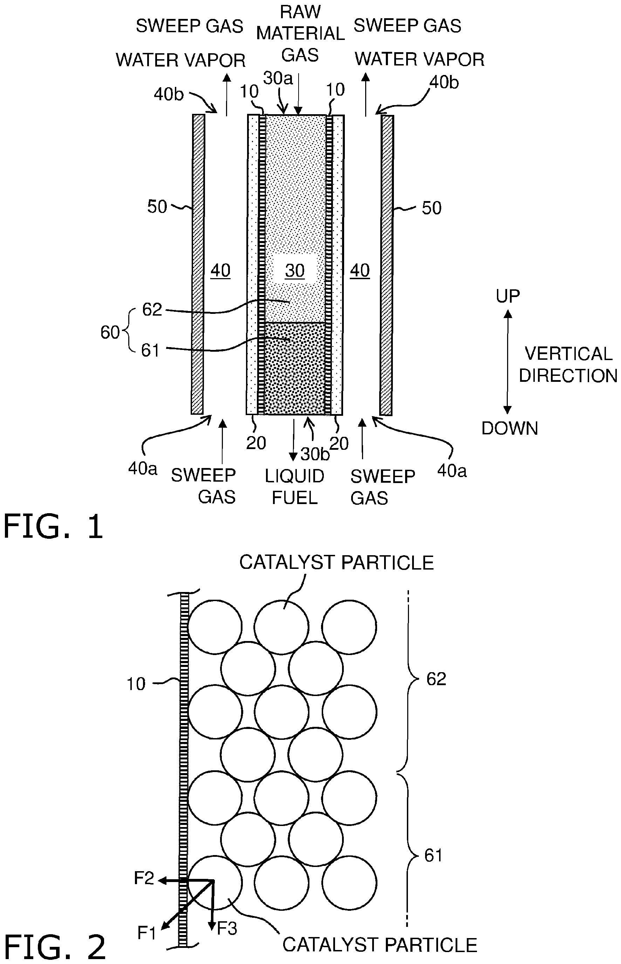

FIG. 1 is a cross-sectional view of a reactor 1.

The reactor 1 is a so-called membrane reactor used to convert a raw material gas to a liquid fuel. The configuration of the reactor 1 can be applied to a fixed bed reactor and a monolith-type reactor.

The raw material gas contains at least hydrogen and carbon oxide. At least one of carbon monoxide and carbon dioxide can be used as the carbon oxide. The raw material gas may be a so-called synthetic gas (syngas).

The liquid fuel is a fuel that is in a liquid state at normal temperature and pressure, or a fuel that can be liquidized at normal temperature and pressurized state. Examples of the liquid fuel that is in a liquid state at normal temperature and pressure may include methanol, ethanol, liquid fuels represented by CₙH₂₍ₘ₋₂ₙ₎ (m is an integer less than 90, and n is an integer less than 30), and mixtures thereof. Examples of the fuel that can be liquidized at normal temperature and pressurized state include, for example, propane, butane, and mixtures thereof.

For example, reaction formula (1) for synthesizing methanol by catalytically hydrogenating a raw material gas containing carbon dioxide and hydrogen in the presence of a catalyst is as follows.

CO₂ + 3H₂ ↔ CH₃OH + H₂O (1)

The above reaction is an equilibrium reaction, and in order to increase both the conversion efficiency and the reaction rate, it is preferable to carry out the reactions at a high temperature and high pressure (for example, 180°C or higher and 2 MPa or higher). The liquid fuel is in a gaseous state when it is synthesized, and is kept in a gaseous state at least until it flows out of the reactor 1. The reactor 1 preferably has heat resistance and pressure resistance suitable for desired synthesis conditions of the liquid fuel.

As shown in FIG. 1, the reactor 1 includes a separation membrane 10, a porous support body 20, a non-permeation side flow path 30, a permeation side flow path 40, an outer tube 50, and a catalyst 60.

The separation membrane 10 is permeable to water vapor, which is one of the products of the conversion reaction of the raw material gas to the liquid fuel. In this manner, by utilizing the equilibrium shift effect, the reaction equilibrium of the above formula (1) can be shifted to the product side. In the present embodiment, the separation membrane 10 is formed in a cylindrical shape.

The separation membrane 10 preferably has a water vapor permeability coefficient of 100 nmol/(s·Pa·m²) or more. The water vapor permeability coefficient can be determined using a known method (see Ind. Eng. Chem. Res., 40, 163-175 (2001)).

The separation membrane 10 preferably has a separation factor of 100 or more. The greater the separation factor is, the more permeable the separation membrane 10 is to water vapor, and the less permeable it is to components other than water vapor (e.g., hydrogen, carbon oxide, and liquid fuel). The separation factor can be determined using a known method (see FIG. 1 of "Separation and Purification Technology 239 (2020) 116533").

The separation membrane 10 is formed in a cylindrical shape. The separation membrane 10 is disposed inward of the cylindrical porous support body 20. The separation membrane 10 is in contact with the inner circumferential face of the porous support body 20.

An inorganic membrane can be used as the separation membrane 10. The inorganic membrane is preferable because it has heat resistance, pressure resistance, and water vapor resistance. Examples of the inorganic membrane include a zeolite membrane, a silica membrane, an alumina membrane, and a composite membrane thereof. In particular, an LTA zeolite membrane having a molar ratio (Si/Al) of a silicon element (Si) and an aluminum element (Al) of 1.0 or more and 3.0 or less is suitable because of its excellent water vapor permeability. Note that the inorganic membrane has a characteristic of being easily broken by thermal shock.

The porous support body 20 is formed in a tubular shape. The porous support body 20 surrounds the separation membrane 10. The porous support body 20 supports the separation membrane 10. The porous support body 20 is constituted by a porous material. In the present embodiment, the porous support body 20 is formed in a cylindrical shape.

As the porous material, a ceramic material, a metal material, a resin material, or the like can be used, and a ceramic material is particularly preferable. As an aggregate of the ceramic material, for example, at least one of alumina (Al₂O₃), titania (TiO₂), mullite (Al₂O₃-SiO₂), potsherd, cordierite (Mg₂Al₄Si₅O₁₈), or the like can be used. As an inorganic binder for the ceramic material, for example, at least one of titania, mullite, readily sinterable alumina, silica, glass frit, a clay mineral, and readily sinterable cordierite can be used. However, the ceramic material does not need to contain an inorganic binder.

The non-permeation side flow path 30 is provided on a non-permeation side of the separation membrane 10. In the present embodiment, the non-permeation side flow path 30 is a columnar space on the inner side of the separation membrane 10.

The non-permeation side flow path 30 extends in an approximately vertical direction. The approximately vertical direction is a concept including not only a direction that matches the gravity direction, but also a direction inclined to some extent (±15°) with respect to the gravity direction.

The raw material gas is caused to flow through the non-permeation side flow path 30. In the present embodiment, the raw material gas is caused to flow downward through the non-permeation side flow path 30. Accordingly, the raw material gas flows in through an upper end opening 30a of the non-permeation side flow path 30, and the liquid fuel flows out through a lower end opening 30b of the non-permeation side flow path 30. Note that the raw material gas may be caused to flow upward through the non-permeation side flow path 30. The liquid fuel flowing out through the lower end opening 30b may be mixed with a residual raw material gas.

The permeation side flow path 40 is provided on the non-permeation side of the separation membrane 10. In the present embodiment, the permeation side flow path 40 is an annular space between the separation membrane 10 and the outer tube 50. Water vapor that has permeated through the separation membrane 10 flows into the permeation side flow path 40.

In the present embodiment, a sweep gas for sweeping water vapor is caused to flow through the permeation side flow path 40. An inert gas (e.g., nitrogen), air, and the like can be used as the sweep gas. In the present embodiment, the sweep gas flows upward (i.e., the opposite direction to the direction in which the raw material gas flows) through the permeation side flow path 40. Accordingly, the sweep gas flows in through the lower end opening 40a of the permeation side flow path 40, and the sweep gas that has taken in water vapor flows out through the upper end opening 40b of the permeation side flow path 40. Note that the sweep gas may be caused to flow downward (i.e., the same direction as the direction in which the raw material gas flows) through the permeation side flow path 40.

The catalyst 60 fills the non-permeation side flow path 30. The catalyst 60 advances (promotes) the conversion reaction of the raw material gas to the liquid fuel. The catalyst 60 is in direct contact with the separation membrane 10.

The catalyst 60 is constituted by a plurality of catalyst particles. The catalyst particles are each constituted by a carrier and a supported catalytic component. As the carrier, for example, alumina, titania, silica, ceria, zeolite or the like can be used, but there is no limitation to this. The supported catalytic component is supported on a surface of the carrier. As the supported catalytic component, metal catalyst component (copper, palladium, and the like), oxide catalyst component (zinc oxide, amorphous zirconia, gallium oxide, and the like), and composite catalyst components thereof can be used, but there is no limitation to this.

The shape of the catalyst particles is not particularly limited, and for example, may be a spherical shape, an ellipsoidal shape, a circular columnar shape, an ellipsoidal columnar shape, a disc-like shape, a scale-like shape, a needle-like shape, a polygonal columnar shape, a sheet-like shape, or the like.

The catalyst 60 has a multilayer structure in which a plurality of layers are stacked in the approximately vertical direction. In the present embodiment, the catalyst 60 is configured as a double-layer structure having a first layer 61 and a second layer 62.

The first layer 61 is located at the lowermost layer of the catalyst 60. The first layer 61 is disposed in the lower end portion of the non-permeation side flow path 30. The lower end portion of the non-permeation side flow path 30 is a region that is lower than the center of the non-permeation side flow path 30 in the approximately vertical direction.

The second layer 62 is disposed upward of the first layer 61. Since the catalyst 60 has the double layer structure in the present embodiment, the second layer 62 is disposed on the first layer 61. The second layer 62 is disposed in a region, of the non-permeation side flow path 30, where the first layer 61 is not disposed.

Here, the mean equivalent circle diameter of the catalyst particles included in the first layer 61 is larger than the mean equivalent circle diameter of the catalyst particles included in the second layer 62. In this manner, damage to the separation membrane 10 due to the weight of the catalyst 60 acting on the separation membrane 10 can be suppressed. The mechanism that can suppress damage to the separation membrane 10 is as follows.

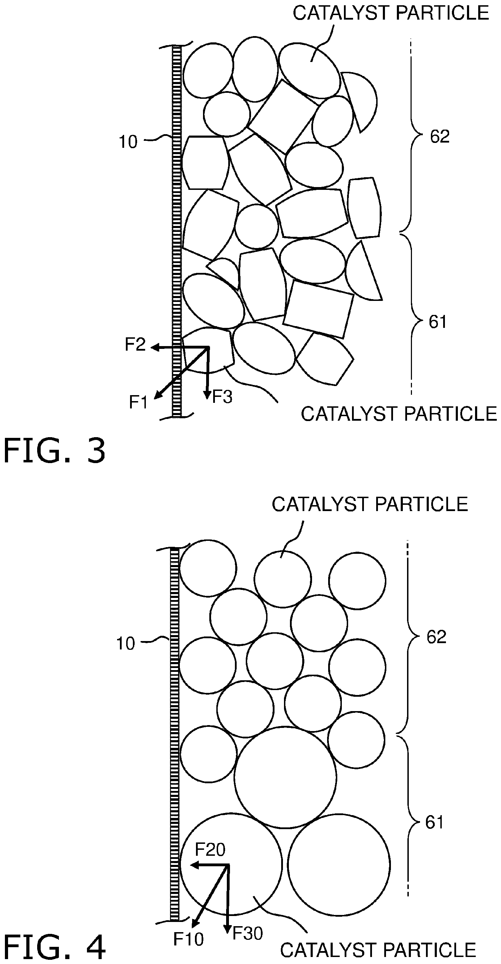

First, FIGS. 2 and 3 schematically show a comparative example in which the mean equivalent circle diameter of the catalyst particles included in the first layer 61 is equal to the mean equivalent circle diameter of the catalyst particles included in the second layer 62. FIG. 2 shows a case where the catalyst particles have a spherical shape, and FIG. 3 shows a case where the catalyst particles have a circular columnar shape. In the modes in FIGS. 2 and 3, since positions of the catalyst particles contained in the first layer 61 and the catalyst particles contained in the second layer 62 are likely to horizontally shift, when a force F 1 acting on the catalyst particles included in the first layer 61 is divided into a horizontal component F2 and a vertical component F3, the horizontal component F2 is large. As a result, a large force acts on the separation membrane 10 by the catalyst particles included in the first layer 61, and the separation membrane 10 is likely to be damaged.

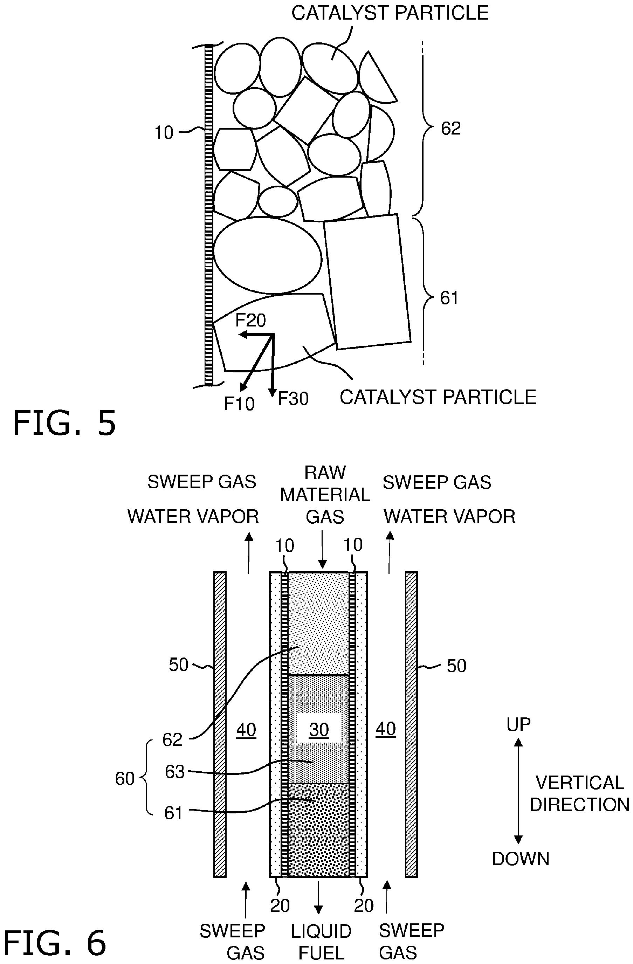

On the other hand, FIGS. 4 and 5 schematically show examples in which the mean equivalent circle diameter of the catalyst particles included in the first layer 61 is larger than the mean equivalent circle diameter of the catalyst particles included in the second layer 62. FIG. 4 shows a case where the catalyst particles have a spherical shape, and FIG. 5 shows a case where the catalyst particles have a circular columnar shape. In the modes in FIGS.4 and 5, since horizontal shifting of the positions of the catalyst particles included in the first layer 61 and the catalyst particles included in the second layer 62 can be suppressed, when a force F10 acting on the catalyst particles included in the first layer 61 is divided into a horizontal component F20 and a vertical component F30, the horizontal component F20 can be made smaller than the horizontal component F2 shown in FIGS. 2 and 3. As a result, a force acting on the separation membrane 10 by the catalyst particles included in the first layer 61 can be reduced, and damage to the separation membrane 10 can be suppressed.

Also, since the mean equivalent circle diameter of the catalyst particles included in the second layer 62 is relatively small, the total surface area can be increased by improving the filling rate of the catalyst particles, and thus a number of supported catalytic components can be supported. As a result, the overall catalytic performance of the catalyst 60 can be ensured.

The mean equivalent circle diameter can be obtained as follows.

First, a resin (e.g., epoxy) is cast in the non-permeation side flow path 30 to be cured, and then the catalyst 60 is cut along the approximately vertical direction.

Next, cross-sectional images of the first layer 61 and the second layer 62 are respectively obtained using a SEM (Scanning Electron Microscope). The magnifying power of the SEM is selected from a range of 1-fold or more and 100-fold or less such that 10 or more (preferably 100 or more) catalyst particles can be observed in one observation field. In this case, when both cross sections of the separation membrane (cross sections on both sides in the horizontal direction of the non-permeation side flow path 30) are not seen in one observation field, the observation field can be divided into two observation fields for observing the cross sections of the separation membrane and one or more observation fields between the two observation fields.

Next, using SEM-EDS (energy dispersive X-ray analysis), elements of the substance exposed on the respective cross sections of the first layer 61 and the second layer 62 are specified, and thereby the catalyst particles are specified on the respective cross-sectional images of the first layer 61 and the second layer 62. Note that the method for specifying the catalyst particles is not limited to the method using the SEM-EDS, and a method using WDS (wavelength dispersive X-ray analysis) may also be adopted, for example.

Next, the arithmetic mean value of the equivalent circle diameters of 30 or more catalyst particles randomly selected from the cross-sectional image of the first layer 61 is obtained, and the arithmetic mean value of the equivalent circle diameters of 30 or more catalyst particles randomly selected from the cross-sectional image of the second layer 62 is obtained. The arithmetic mean value of the first layer 61 obtained as above is the mean equivalent circle diameter of the catalyst particles included in the first layer 61, and the arithmetic mean value of the second layer 62 is the mean equivalent circle diameter of the catalyst particles included in the second layer 62. Note that the equivalent circle diameter of the catalyst particle means the diameter of a circle having the same area as a catalyst particle.

Although there is no limitation on the value of the mean equivalent circle diameter of the catalyst particles included in the first layer 61, the value can be 1000 µm or more and 10000 µm or less, for example. Note that if the mean equivalent circle diameter of the catalyst particles in the first layer 61 is set small, variation in the pressure loss increases, which incurs uneven flow of the gas. As such, the mean equivalent circle diameter of the catalyst particles in the first layer 61 is preferably 500 µm or more. Also, tension stress is likely to act on the separation membrane 10 provided on the inner side of the porous support body 20. As such, by setting the mean equivalent circle diameter of the catalyst particles in the first layer 61 to 500 µm or more, the stress acting on the separation membrane 10 can be suppressed.

Although there is no limitation on the value of the mean equivalent circle diameter of the catalyst particles included in the second layer 62, the value can be 6000 µm or less, for example. Note that if the mean equivalent circle diameter of the catalyst particles in the second layer 62 is set small, variation in the pressure loss increases, which incurs uneven flow of the gas. As such, the mean equivalent circle diameter of the catalyst particles in the second layer 62 is preferably 500 µm or more. Also, tension stress is likely to act on the separation membrane 10 provided on the inner side of the porous support body 20. As such, by setting the mean equivalent circle diameter of the catalyst particles in the second layer 62 to 500 µm or more, the stress acting on the separation membrane 10 can be suppressed.

The catalyst 60 can be formed by putting the catalyst particles for the first layer 61 into the non-permeation side flow path 30 from above, and then putting the catalyst particles for the second layer 62 into the non-permeation side flow path 30 from above.

### Modification of embodiment

Although an embodiment of the present invention has been described above, the present invention is not limited to the above embodiment, and various modifications can be made without departing from the gist of the invention.

### Modification 1

Although the inner side of the separation membrane 10 is the non-permeation side flow path 30 and the outer side of the separation membrane 10 is the permeation side flow path 40 in the above embodiment, a configuration is also possible in which the outer side of the separation membrane 10 is the non-permeation side flow path 30 and the inner side of the separation membrane 10 is the permeation side flow path 40. In this case, it is preferable that the separation membrane 10 is formed surrounding the outer side of the porous support body 20.

### Modification 2

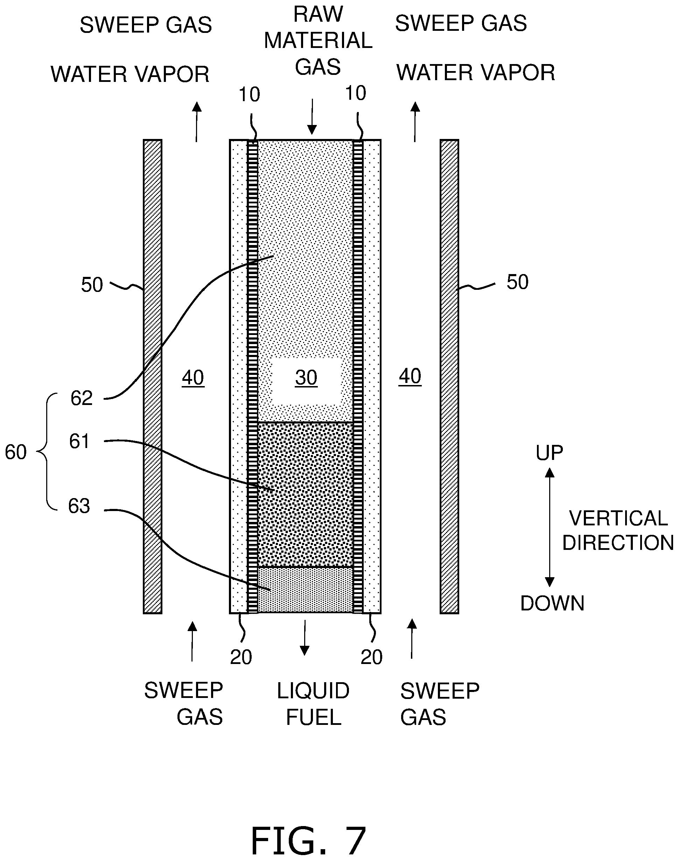

Although the catalyst 60 has a double-layer structure having the first layer 61 and the second layer 62 in the above embodiment, the catalyst 60 may have a structure that includes three or more layers. For example, as shown in FIGS. 6 and 7, the catalyst 60 may have a triple layer structure that includes a third layer 63 in addition to the first layer 61 and the second layer 62.

In FIG. 6, the third layer 63 is disposed between the first layer 61 and the second layer 62 in the approximately vertical direction. It is preferable that the mean equivalent circle diameter of the catalyst particles included in the third layer 63 is smaller than the mean equivalent circle diameter of those included in the first layer 61 and larger than the mean equivalent circle diameter of the catalyst particles included in the second layer 62. In this manner, since a force acting on the separation membrane 10 by the catalyst particles included in the first layer 61 can be further reduced, damage to the separation membrane 10 can be further suppressed. Also, since a force acting on the separation membrane 10 by the catalyst particles included in the third layer 63 can also be further reduced, the range in which damage to the separation membrane 10 can be suppressed can be made wider.

Note that other layers may be disposed between the first layer 61 and the third layer 63, and between the second layer 62 and the third layer 63. It is preferable that the mean equivalent circle diameter of the catalyst particles included in such layers are larger in the lower layer and smaller in the upper layer.

In FIG. 7, the third layer 63 is disposed below the first layer 61 in the approximately vertical direction. The third layer 63 includes at least one of the catalyst particles and the filler particles. The filler particles need not include catalytic function. The filler particles can be constituted by, for example, ceramic material (alumina, titania, silica, ceria, zeolite, or the like). In the case where the third layer 63 includes the catalyst particles, the mean equivalent circle diameter of the catalyst particles included in the third layer 63 may be larger than, smaller than, or the same as the mean equivalent circle diameter of the catalyst particles included in the first layer. Also, in the case where the third layer 63 includes the filler particles, the mean equivalent circle diameter of the filler particles included in the third layer 63 may be larger than, smaller than, or the same as the mean equivalent circle diameter of the catalyst particles included in the first layer.

In this manner, even in the case where the first layer 61 is not disposed at the lowermost layer, damage to a portion of the separation membrane 10 that is in contact with the first layer 61 can be suppressed. Note that in a case where the mean equivalent circle diameter of the catalyst particles or the filler particles included in the third layer 63 is small, a portion of the separation membrane 10 that is in contact with the third layer 63 may be damaged, and thus it is preferable that the first layer 61 is disposed as the lowermost layer.

### Modification 3

Although the separation membrane 10 is permeable to water vapor that is a product of the conversion reaction of the raw material gas to the liquid fuel in the above embodiment, the present invention is not limited thereto. The separation membrane 10 may be permeable to the liquid fuel itself, which is a product of the conversion reaction of the raw material gas to the liquid fuel. In this case as well, the reaction equilibrium of the above formula (1) can be shifted to the product side.

Also, when the separation membrane 10 is permeable to the liquid fuel, even when generating the liquid fuel through a reaction in which no water vapor is generated (e.g., 2H₂ + CO ↔ CH₃OH), the reaction equilibrium can be shifted to the product side.

### REFERENCE SIGNS LIST

- 1: Reactor
- 10: Separate membrane
- 20: Porous support body
- 30: Non-permeation side flow path
- 30a: Upper end opening
- 30b: Lower end opening
- 40: Permeation side flow path
- 40a: Lower end opening
- 40b: Upper end opening
- 50: Outer tube
- 60: Catalyst
- 61: First layer
- 62: Second layer
- 63: Third layer

## Claims

1. A reactor comprising:
a separation membrane permeable to a product of a conversion reaction of a raw material gas containing at least hydrogen and carbon oxide to a liquid fuel;
a non-permeation side flow path extending in an approximately vertical direction on a non-permeation side of the separation membrane, the raw material gas flowing through the non-permeation side flow path; and
a catalyst configured to fill the non-permeation side flow path and promote the conversion reaction,
the catalyst including a first layer and a second layer disposed upward of the first layer, and
a mean equivalent circle diameter of catalyst particles included in the first layer is larger than a mean equivalent circle diameter of catalyst particles included in the second layer.

2. The reactor according to claim 1, wherein
the first layer is located at a lowermost layer of the catalyst.
